Europäisches Patentamt

European Patent Office

Office européen des brevets

(11) Veröffentlichungsnummer: **0 405 148 A1**

(12) # EUROPÄISCHE PATENTANMELDUNG

(21) Anmeldenummer: 90110021.4

(51) Int. Cl.5: **A61M 5/142**, A61M 5/172

(22) Anmeldetag: 26.05.90

(30) Priorität: 30.06.89 DE 3921555
20.12.89 DE 3942110

(43) Veröffentlichungstag der Anmeldung:
02.01.91 Patentblatt 91/01

(84) Benannte Vertragsstaaten:
AT BE CH DE DK ES FR GB IT LI NL SE

(71) Anmelder: **B. Braun Melsungen AG**
**Carl-Braun Strasse**
**D-3508 Melsungen(DE)**

(72) Erfinder: **Heitmeier, Rolf**
**Goethestrasse 8**
**D-3507 Baunatal(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Infusionsvorrichtung.**

(57) Die Infusionsvorrichtung weist einen Flüssig-keitsbehälter (10), eine Pumpe (12) und einen zum Patienten führenden Schlauch (14) auf. In den Schlauch (14) ist ein Strömungssensor (17) inte-griert, der das Vorhandensein von Flüssigkeitsströ-mung im Schlauch (14) erkennt. Dieser Strömungs-sensor (17) ist ein Thermo-Strömungssensor mit ei-nem Widerstandselement, dessen Strom so geregelt ist, daß die zugeführte Stromwärme stets gleich der von der Flüssigkeit abgeführten Wärme ist. Bei einer Schlauchokklusion wird das Aussetzen der Flüssig-keisttrömung erkannt und Alarm erzeugt. Der Strö-mungssensor (17) ist möglichst nahe am Patienten plaziert und kurz vor dem Katheteranschluß (15) angeordnet.

FIG.1

## INFUSIONSVORRICHTUNG

Die Erfindung betrifft eine Infusionsvorrichtung der im Oberbegriff des Patentanspruchs 1 angegebenen Art.

Bei der Zuführung von Infusionslösungen, die genau zu dosierende Medikamentenzusätze enthalten, zu Patienten, sowie bei der unmittelbaren Zufuhr hochpotenter Medikamente und bei der parenteralen Ernährung werden Infusionspumpen benutzt, die die Flüssigkeit dem Patienten in hochgenauer Dosierung zuführen. Solche Infusionspumpen können als Schlauchpumpen oder auch als Spritzenpumpen ausgebildet sein. Die Infusionspumpen müssen einen Druck aufbringen, der größer ist als der Druck, der sich aus den Strömungswiderständen des Flüssigkeitsleitungssystems und dem Gegendruck des Patienten zusammensetzt. Sie müssen ferner imstande sein, eine vorgewählte Infusionsrate mit hoher Genauigkeit einzuhalten. In der Praxis werden von Infusions pumpen Drücke bis zu 1,0 bar erzeugt. Um sicherzustellen, daß der Pumpendruck keinen zu hohen Wert annimmt, ist es bekannt, Druckbegrenzungseinrichtungen vorzusehen. Eine bekannte Druckbegrenzungseinrichtung weist ein elastisches Schlauchstück in der Infusionsleitung auf, das sich entsprechend dem Flüssigkeitsdruck aufweitet. Diese Aufweitung wird von einem optischen oder mechanischen Wächter erkannt, der feststellt, ob der Schlauchdurchmesser ein bestimmtes Maß überschritten hat. Wenn im Schlauch zwischen Pumpe und Patient eine Okklusion, z.B. durch Abknicken des Schlauchs, stattfindet, erhöht sich der im Schlauch herrschende Druck, wodurch der Wächter anspricht und Alarm erzeugt und/oder die Pumpe abschaltet.

Bei kleineren Arbeitsdrücken der Pumpe und bei plötzlich auftretenden Okklusionen ergibt sich der Nachteil, daß bis zum Erreichen des Druckes, der zur Alarmerzeugung oder zum Abschalten der Pumpe führt, eine große Zeitspanne verstreicht. Während dieser Zeit ist die Medikamentenzufuhr zum Patienten unterbrochen, ohne daß dem Anwender der Infusionsvorrichtung dies mitgeteilt wird. Ein weiterer Nachteil besteht darin, daß sich in dem elastischen Schlauchstück bei Überdruck ein erhebliches Bolusvolumen ansammelt, das sich bei Aufhebung (Beendigung) der Okklusion zum Patienten hin entlädt, so daß der Patient stoßweise einen Medikamentenbolus erhält.

Nach dem Stand der Technik erfolgt die Okklusionserkennung somit indirekt und mit einer erheblichen Zeitverzögerung durch den sich erhöhenden Druck im Schlauchsystem. Bei praktischen Ausführungen beträgt die Reaktionszeit ca. 10 Minuten und das sich im elastischen Schlauchstück aufbauende Bolusvolumen beträgt ca. 1 ml. Die bekannten Wächter zum Überwachen eines Infusionssystems auf Okklusion haben insbesondere Nachteile auch bei Mehrfachinfusionen, bei denen zwei Pumpen, die unterschiedliche Flüssigkeiten zuführen, gemeinsam zu einer Verbindungsstelle fördern, von der ein Schlauch zum Patienten führt, wobei zwischen jeder der Pumpen und der Verbindungsstelle ein Wächter vorhanden ist. Ist die eine Pumpe abgeschaltet und tritt in der von der Verbindungsstelle zum Patienten führenden Schlauchleitung eine Okklusion auf, dann fördert die laufende Pumpe über die stillstehende Pumpe Flüssigkeit in das mit der stillstehenden Pumpe verbundene Vorratsgefäß. Dort werden beide Flüssigkeiten miteinander vermischt, ohne daß dem Patienten Flüssigkeit zugeführt wird. Diese Situation bleibt dem Anwender verborgen, da kein Alarm erzeugt wird.

Eine Infusionsvorrichtung, von der der Oberbegriff des Patentanspruchs 1 ausgeht, ist bekannt aus DE 30 18 641 C2. Bei dieser Infusionsvorrichtung sind an dem Schlauch eine Mengenmeßeinrichtung und eine Druckmeßeinrichtung vorgesehen, um Schlauchokklusionen feststellen zu können. Alarm wird erzeugt, wenn der Druck in der Druckmeßeinrichtung null und die Durchflußmenge null ist oder wenn die Durchflußmenge null und der Druck hoch ist oder wenn der Druck null und die Durchflußmenge hoch ist. Über die Art des verwendeten Durchflußmessers sind keine Angaben gemacht. Da Durchfluß und Druck beide ausgewertet werden und da die Druckmeßvorrichtung wegen der Aufweitung des elastischen Schlauchs mit großer Verzögerung anspricht, können Schlauchokklusionen erst sehr spät erkannt werden, wenn der Patient über längere Zeit hinweg nicht mit dem Medikament versorgt wurde. Ein weiterer Nachteil be steht darin, daß die Meßeinrichtung das Vorhandensein von Gasbläschen, z.B. Lufteinschlüssen, im Leitungssystem nicht erkennt. Normalerweise sind für das Erkennen von Gasbläschen zusätzliche Erkennungseinrichtungen erforderlich.

Der Erfindung liegt die Aufgabe zugrunde, eine Infusionsvorrichtung der im Oberbegriff des Patentanspruchs 1 angegebenen Art zu schaffen, bei dem in kurzer Zeit Schlauchokklusionen sowie auch das Vorhandensein von Luft erkannt wird, um eine Patientengefährdung auszuschließen.

Die Lösung dieser Aufgabe erfolgt erfindungsgemäß mit den im kennzeichnenden Teil des Patentanspruchs 1 angegebenen Merkmalen.

Bei der erfindungsgemäßen Infusionsvorrichtung weist der Strömungswächter einen Thermo-Strömungssensor mit Heizelement auf. Wenn Schlauchokklusion auftritt, bleibt die Strömungs-Wärmeabfuhr vom Heizelement aus, so daß das Heizelement sich stärker erwärmt als bei Normalbetrieb. Dieser Umstand wird erkannt und für die Alarmerzeugung ausgenutzt. Eine stärkere Erhöhung der Temperatur des Heizelementes ergibt sich auch, wenn sich in der Nähe des Heizelementes wärmeisolierende Gasblasen (Luftblasen) befinden. Das Vorhandensein von Gasblasen wird also mit derselben Erkennungseinrichtung und in gleicher Weise erkannt wie das Ausbleiben der Flüssigkeitsströmung. Der Strömungswächter reagiert sowohl auf Okklusionen als auch auf Gasblasen relativ schnell, weil in dem elastischen Schlauch kein Druckaufbau erforderlich ist. Der Patient wird nach Beseitigung einer Schlauchokklusion nicht mit einem schockartigen Medikamentenbolus beaufschlagt.

Der Thermo-Strömungssensor bzw. dessen Heizelement, braucht nicht unmittelbar in der Strömung angeordnet zu sein. Es genügt, daß er sich in wärmeleitendem Kontakt mit der Strömung befindet. Beispielsweise kann der Schlauch einen kurzen Rohrabschnitt aus wärmeleitendem Metall aufweisen, an dem das Heizelement befestigt ist.

Infusionsvorrichtungen sind Einmalartikel, die nach Gebrauch fortgeworfen werden. Solche Einmalartikel sollten möglichst einfach und kostengünstig herstellbar sein und möglichst wenig oder keine bewegbaren Teile enthalten. Da der Strömungssensor im Verlauf des Einmal-Systems vorgesehen ist, sollte er ebenfalls einfach und kostengünstig herstellbar sein. Dieses Heizelement wird durch den Strom einer Stromquelle aufgeheizt und die an ihm entlangströmende Flüssigkeit führt Stromwärme ab. Die Stromquelle ist so geregelt, daß ein thermisches Gleichgewicht eintritt, d.h. daß die zugeführte Stromwärme gleich der durch die Flüssigkeit abgeführten Wärme ist. Das Ausgangssignal der Stromquelle gibt Aufschluß über denjenigen Strom, der erforderlich ist, um die durch die Flüssigkeit hervorgerufene Wärmeabfuhr zu kompensieren. Wenn die Flüssigkeit stillsteht, ist die Wärmeabfuhr gering, so daß die Stromquelle nur einen geringen Heizstrom liefert. Wenn der Heizstrom einen Referenzwert unterschreitet, wird angenommen, daß keine Flüssigkeitsströmung vorhanden ist und daß Alarm erzeugt werden muß.

Der Thermo-Strömungssensor kann in den zum Einmal-System gehörenden Schlauch integriert sein. Es handelt sich um ein relativ einfach herzustellendes Bauteil, das als Dickschicht-Bauelement ausgeführt werden kann. Die Auswerteschaltung, die an den Thermosensor angeschlossen wird, ist Bestandteil eines Gerätes, das nicht zum Einmal-System gehört und das lediglich über zwei Drähte mit dem Thermosensor verbunden wird.

Vorzugsweise ist der Thermosensor in der Schlauchleitung möglichst nahe am Patienten plaziert. Er sollte am patientenseitigen Ende des Schlauchs kurz vor dem Katheteranschlußstück oder im Inneren dieses Katheteranschlußstücks angeordnet sein.

Andererseits ist es auch möglich, den erfindungsgemäßen Strömungswächter mit Thermo-Strömungssensor anstelle der üblichen Tropfsysteme zu verwenden, die zwischen dem Vorratsgefäß und der Pumpe angeordnet sind. An dieser Stelle kann der Thermo-Strömungswächter zur Erkennung der Tatsache, daß das Vorratsgefäß leergelaufen ist, verwendet werden. Schließlich eignet sich der Thermo-Strömungssensor auch zur Luftblasenerkennung in Infusionssystemen, weil bei Vorhandensein von Gasblasen in der Flüssigkeit die Wärmeabfuhr vom Widerstandselement verringert ist.

Im folgenden wird unter Bezugnahme auf die Zeichnungen ein Ausführungsbeispiel der Erfindung näher erläutert.

Es zeigen:
Fig. 1 eine schematische Gesamtdarstellung der Infusionsvorrichtung,
Fig. 2 eine Darstellung der elektrischen Schaltung und
Fig. 3 einen Schnitt durch den Schlauch an der Stelle III von Fig. 1, an der sich der Strömungssensor befindet.

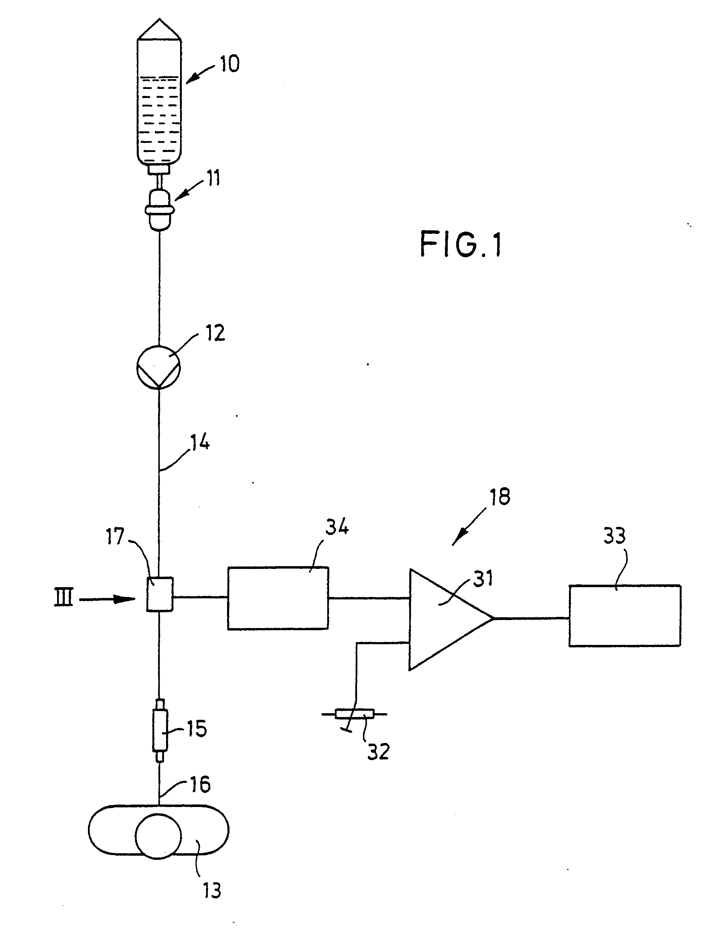

Das in Fig. 1 dargestellte Infusionssystem weist ein Vorratsgefäß 10 auf, in dem sich die Infusionsflüssigkeit befindet. Aus diesem Vorratsgefäß 10 läuft die Flüssigkeit zunächst in eine Tropfkammer 11 und von dort zu einer Pumpe 12. Die Pumpe 12 fördert die Flüssigkeit mit vorgegebener Infusionsrate in einen zum Patienten 13 führenden Schlauch 14. Am Ende dieses Schlauchs 14 befindet sich das Katheteranschlußstück 15, das mit dem Katheter 16 verbunden werden kann, welcher zuvor in den Körper des Patienten eingeführt worden ist.

Unmittelbar vor dem Katheteranschlußstück 15 ist im Kanal des Schlauches 14 der Strömungssensor 17 angeordnet, der Bestandteil des Strömungswächters 18 ist.

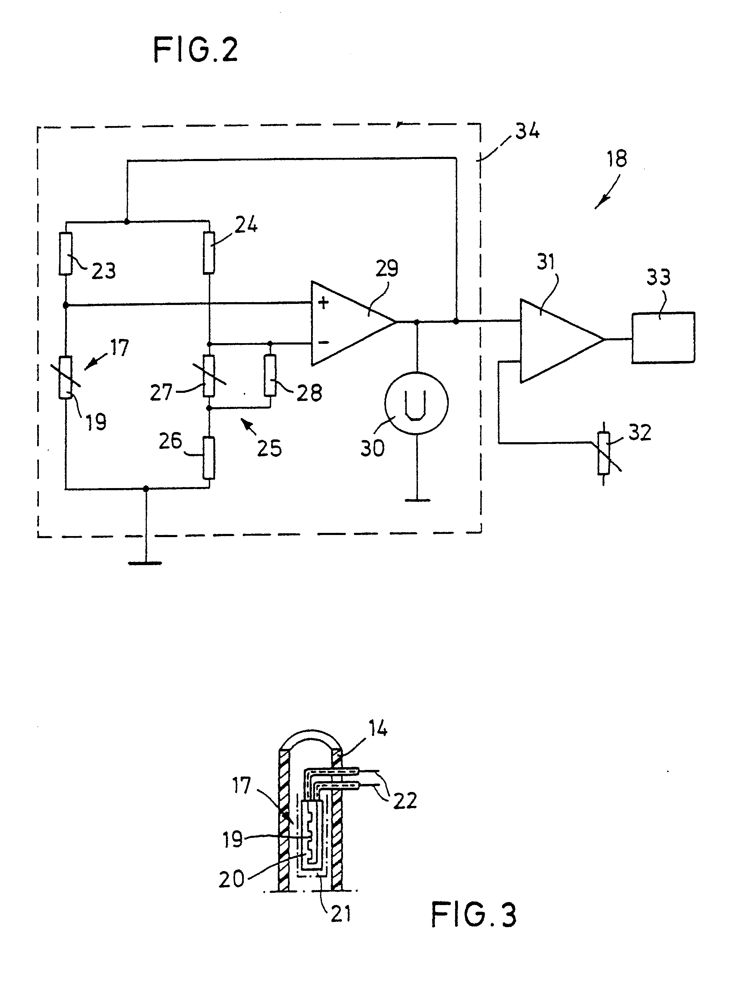

Wie aus Fig. 3 zu erkennen ist, weist der Strömungssensor 17 ein Heizdraht-Widerstandselement 19 auf, das als Leiterbahn auf einem Keramiksubstrat 20 in Dickschichttechnik ausgeführt ist. Das Widerstandselement ist mit einem gut wärmeleitenden, elektrisch isolierenden Überzug 21 versehen. Es kann in die Wand des Schlauchs 14 eingelassen oder lose in den Schlauch eingehängt sein. Von den beiden Enden des Widerstandselements 19 führen isolierte Drähte 22 aus dem Schlauch 14 heraus.

Wie Fig. 2 zeigt, ist das Widerstandselement 19 Bestandteil einer Brückenschaltung. Das Widerstandselement 19 liegt in Reihe mit einem Widerstand 23 und bildet mit diesem den ersten Brük-

kenzweig. Der zweite Brückenzweig besteht aus der Reihenschaltung eines Widerstandes 24 und einer Widerstandsanordnung 25. Die Widerstandsanordnung 25 enthält einen Reihenwiderstand 26 und die Parallelschaltung eines Thermistors 27 und eines weiteren Widerstandes 28.

Die beiden Punkte des Brückennullzweiges sind an die Eingänge eines Differenzverstärkers angeschlossen, der die Stromquelle 29 bildet. Der Ausgang der Stromquelle 29 speist das eine Ende der beiden Brückenzweige, während das andere Ende an Massepotential liegt.

Der Thermistor 27 dient der Temperaturkompensation. Er ist in gleicher Weise in dem Schlauch angeordnet wie der Strömungssensor 17.

Das Ausgangssignal der Stromquelle 29 wird von einem Voltmeter 30 angezeigt und außerdem einem Schwellwertverstärker 31 zugeführt, dessen Schwellenwert an einem Widerstand 32 veränderbar ist. Das Ausgangssignal des Schwellwertverstärkers 31 steuert eine Alarmeinrichtung 33. Die Brückenschaltung bildet zusammen mit der Stromquelle 29 die Meßschaltung 34.

Der Strömungswächter arbeitet wie folgt: Die Stromquelle 29 erzeugt auch dann eine Ausgangsspannung von vorbestimmter Größe, wenn sich die Brückenschaltung im Gleichgewichtszustand befindet. Diese Ausgangsspannung wird erhöht, wenn der Widerstandswert des Widerstandselements 19 infolge starker Strömung abnimmt und sie wird verringert, wenn der Widerstandswert des Widerstandselementes 19 infolge verringerter Flüssigkeitsströmung steigt. Wenn in dem Kanal des Schlauchs 14 Flüssigkeitsströmung vorhanden ist, führt die strömende Flüssigkeit von dem Strömungssensor 17 Wärme ab. Dadurch verringert sich der Widerstandswert des Widerstandselementes 19 und die Meßbrücke gerät ins Un gleichgewicht, so daß zwischen den Eingängen der Stromquelle 29 eine Spannungsdifferenz entsteht. Die Stromquelle 29 liefert daraufhin erhöhten Strom an die Meßbrücke, so daß der Strom in beiden Brückenzweigen erhöht wird. Daraufhin vergrößert sich infolge der zunehmenden Erwärmung des Widerstandselements 19 der Widerstandswert dieses Widerstandselements, bis die Brückenschaltung wieder im Gleichgewicht ist. Die Stromquelle 29 ist also so geregelt, daß die Stromwärme, die vom Widerstandselement 19 erzeugt wird, gleich derjenigen Wärmemenge ist, die durch die strömende Flüssigkeit vom Widerstandselement abgeführt wird. Das Ausgangssignal der Stromquelle 29 wird an dem Voltmeter 30 angezeigt, so daß der Anwender eine Information über die Größe der Strömung erhält.

An dem Potentiometer 32 wird die Untergrenze der Strömungsrate eingestellt. Wenn das Ausgangssignal der Stromquelle 29 unter diese Untergrenze absinkt, erzeugt der Schwellwertverstärker 31 ein Ausgangssignal, durch das die Alarmeinrichtung 33 betätigt wird.

## Ansprüche

1. Infusionsvorrichtung mit einer Pumpe (12), einem an den Ausgang der Pumpe anschließbaren Schlauch (14) und einem im Bereich des Schlauchs (14) angeordneten Strömungswächter (18) mit Strömungssensor (17) zur Ermittlung von Schlauchokklusionen,
**dadurch gekennzeichnet,**
daß der Strömungssensor (17) ein Thermo-Strömungssensor mit einem mit der strömenden, wärmeabführenden Flüssigkeit in thermischem Kontakt stehenden Heizelement (19) ist und daß eine auf die Wärmeabfuhr von dem Heizelement (19) ansprechende Auswerteschaltung (34) vorgesehen ist, die bei nicht strömender Flüssigkeit oder bei Anwesenheit wärmeisolierender Gasblasen in der Umgebung des Heizelementes ein Ausgangssignal zur Betätigung einer Alarmeinrichtung erzeugt.

2. Infusionsvorrichtung nach Anspruch 1, dadurch gekennzeichnet, daß die Auswerteschaltung (34) eine Stromquelle (29) aufweist, die das Heizelement (19) speist und die derart geregelt ist, daß der Spannungsabfall am Heizelement (19) unabhängig von temperaturbedingten Widerstandsänderungen konstant bleibt, und daß die Stromquelle (29) das Ausgangssignal liefert.

3. Infusionsvorrichtung nach Anspruch 1, dadurch gekennzeichnet, daß der Strömungssensor (17) unmittelbar vor oder in einem Katheteranschlußstück (15) des Schlauchs (14) angeordnet ist.

4. Infusionsvorrichtung nach einem der Ansprüche 1 bis 3, dadurch gekennzeichnet, daß der Strömungs sensor (17) in einem Längszweig einer von der Stromquelle (29) gespeisten Brückenschaltung liegt.

5. Infusionsvorrichtung nach Anspruch 4, dadurch gekennzeichnet, daß der andere Längszweig der Brückenschaltung einen Temperaturkompensationswiderstand (27) enthält, der ebenfalls in wärmeleitendem Kontakt mit der Flüssigkeit steht.

6. Infusionsvorrichtung nach einem der Ansprüche 3 bis 5, dadurch gekennzeichnet, daß der Strömungssensor (17) zwischen einem Flüssigkeitsbehälter (10) und der Pumpe (12) angeordnet ist.

# FIG.1

# FIG.2

# FIG.3

## EINSCHLÄGIGE DOKUMENTE

EP 90110021.4

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| D,Y | <u>DE - C2 - 3 018 641</u><br>(RODLER, HANS)<br>* Ansprüche 1-3; Fig. 1 * | 1 | A 61 M 5/142<br>A 61 M 5/172 |
| | -- | | |
| Y | <u>EP - A1 - 0 289 361</u><br>(MEDISTRON LTD.)<br>* Spalte 8, Zeilen 36 ff;<br>Ansprüche 1-3 * | 1 | |
| A | * Spalte 8, Zeilen 36 ff;<br>Ansprüche 1-3 * | 2 | |
| | -- | | |
| A | <u>WO - A1 - 87/04 079</u><br>(SAWYER, PHILIP et al.)<br>* Seite 16, Zeilen 14 ff;<br>Fig. 3 * | 3 | |
| | -- | | |
| A | <u>DE - A1 - 3 702 623</u><br>(DEGUSSA AG)<br>* Spalte 1, Zeilen 30 ff;<br>Fig. * | 4,5 | |
| | -- | | |
| A | <u>WO - A1 - 86/03 415</u><br>(BAXTER TRAVENOL LABORATORIES INC.)<br>* Zusammenfassung; Fig. 1 *<br>---- | 6 | **RECHERCHIERTE SACHGEBIETE (Int Cl⁵)**<br><br>A 61 M 5/00<br>G 01 F 1/00 |

Der vorliegende Recherchenbericht wurde für alle Patentansprüche erstellt

| Recherchenort<br>WIEN | Abschlußdatum der Recherche<br>06-09-1990 | Prüfer<br>SCHNEEMANN |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument